(19) Europäisches Patentamt
European Patent Office
Office européen des brevets

(11) **EP 2 326 275 B1**

(12) **EUROPÄISCHE PATENTSCHRIFT**

(45) Veröffentlichungstag und Bekanntmachung des Hinweises auf die Patenterteilung:
**08.08.2018  Patentblatt 2018/32**

(21) Anmeldenummer: **09778351.8**

(22) Anmeldetag: **04.09.2009**

(51) Int Cl.:
*B25J 3/04* *(2006.01)*          *B25J 19/00* *(2006.01)*
*A61B 34/37* *(2016.01)*          *A61B 34/35* *(2016.01)*
*A61B 34/00* *(2016.01)*

(86) Internationale Anmeldenummer:
**PCT/EP2009/006454**

(87) Internationale Veröffentlichungsnummer:
**WO 2010/025943 (11.03.2010 Gazette 2010/10)**

(54) **MEDIZINISCHER ARBEITSPLATZ UND BEDIENVORRICHTUNG ZUM MANUELLEN BEWEGEN EINES ROBOTERARMS**

MEDICAL WORKSTATION AND OPERATING DEVICE FOR THE MANUAL MOVEMENT OF A ROBOT ARM

POSTE DE TRAVAIL MÉDICAL ET DISPOSITIF DE COMMANDE POUR LE DÉPLACEMENT MANUEL DU BRAS D UN ROBOT

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **08.09.2008   DE 102008041867**

(43) Veröffentlichungstag der Anmeldung:
**01.06.2011   Patentblatt 2011/22**

(73) Patentinhaber: **KUKA Deutschland GmbH 86165 Augsburg (DE)**

(72) Erfinder: **ORTMAIER, Tobias 30966 Hemmingen (DE)**

(74) Vertreter: **Ege Lee & Partner Patentanwälte PartGmbB Schirmgasse 268 84028 Landshut (DE)**

(56) Entgegenhaltungen:
**WO-A-01/18617          WO-A-2007/005367
WO-A-2007/047782     US-A- 4 000 819
US-A1- 2004 034 302     US-A1- 2008 188 868**

**Beschreibung**

Die Erfindung betrifft einen medizinischen Arbeitsplatz und eine Bedienvorrichtung zum manuellen Bewegen eines Roboterarms.

In der Telepräsenz bzw. Teleaktion werden Roboter über relativ große Distanzen ferngesteuert. Dabei werden Sollkommandos von einer Bedienperson an einer Bedienvorrichtung bzw. Eingabekonsole sensoriell erfasst, verarbeitet und an den entfernt stehenden Roboter übertragen. Gegebenenfalls über einen visuellen Rückkanal kann die Bewegung des Roboters überwacht werden. Personen weisen jedoch im Allgemeinen ein gewisses Zittern, den so genannten Tremor auf, wodurch die gewollte manuelle Bewegung der Bedienvorrichtung durch eine ungewollte durch den Tremor erzeugte Zitterbewegung überlagert wird. Dieser Effekt wird noch verstärkt, wenn ein Hochskalieren der Bewegung an der Bedienvorrichtung realisiert ist, also die Bewegungen des Roboters größer als die der Eingabestation sind.

Die EP 0 883 376 B1 offenbart einen medizinischen Arbeitsplatz mit mehreren zum Behandeln eines Patienten vorgesehenen Roboterarmen, die mittels einer Bedienvorrichtung des medizinischen Arbeitsplatzes manuell bewegt werden können. Die Bedienvorrichtung umfasst eine Steuereinrichtung und mit dieser gekoppelte manuell bewegbare erste und zweite Eingabeeinrichtungen. Werden die Eingabeeinrichtungen bewegt, so erzeugt die Steuereinrichtung ein Signal, um die Roboterarme entsprechend zu bewegen. Die Steuereinrichtung weist ferner ein analoges oder digitales Filter auf, das aus dem Signal eine einem Hand-Tremor des die Bedienvorrichtung bedienenden Chirurgen zugeordneten Signalkomponente im Frequenzbereich von 6-12 Hz herausfiltert.

Die WO 2007/005367 A2 offenbart eine Bedienvorrichtung zum manuellen Bewegen eines Roboterarms, negative Auswirkungen eines Tremors der die Bedienvorrichtung bedienenden Person und eine Filterung, um diese negativen Auswirkungen zu kompensieren.

Die Aufgabe der Erfindung ist es, eine alternative Bedienvorrichtung für einen solchen medizinischen Arbeitsplatzes anzugeben, die relativ unempfindlich gegen einen Tremor einer die Bedienvorrichtung bedienenden Person ist.

Die Aufgabe der Erfindung wird gelöst durch eine Bedienvorrichtung gemäß dem Patentanspruch 1.

Die erfindungsgemäße Bedienvorrichtung ist dafür vorgesehen, den Roboterarm über eine gewisse Entfernung manuell zu bewegen. Die mechanische Dämpfungsvorrichtung kann insbesondere derart eingerichtet sein, dass sie beim manuellen Bewegen der Eingabevorrichtung eine der manuellen Bewegung entgegen gerichtete Kraft und/oder ein der manuellen Bewegung entgegen gerichtetes Drehmoment zum zumindest teilweise Unterdrücken einer durch einen Tremor der die Eingabevorrichtung bedienenden Person resultierenden Teilbewegung erzeugt. Eine mögliche Anwendung für die erfindungsgemäße Bedienvorrichtung ist im medizinischen Umfeld, in dem z.B. ein Arzt ein Lebewesen mittels eines oder mehrerer Roboterarme behandelt. Ein weiterer Aspekt der Erfindung betrifft daher einen medizinischen Arbeitsplatz, der die erfindungsgemäße Bedienvorrichtung und wenigstes einen zum Behandeln des Lebewesens vorgesehenen medizinischen Roboterarm aufweist, dessen Bewegung mittels der Bedienvorrichtung manuell steuerbar ist.

Wird die erfindungsgemäße Bedienvorrichtung im medizinischen Umfeld eingesetzt, dann kann der wenigstens eine Roboterarm beispielsweise mit einem medizinischen Instrument, insbesondere mit einem minimalinvasiven medizinischen Instrument versehen sein, wobei der Arzt den Roboterarm und somit das medizinische Instrument mittels der manuellen Eingabevorrichtung bewegt. Der Roboterarm kann beispielsweise in sechs Freiheitsgraden bewegt werden. Die Eingabevorrichtung kann z.B. dieselbe Anzahl von Freiheitsgraden wie oder mehr Freiheitsgrade als der zu bewegende Roboterarm aufweisen. Aufgrund des manuellen Bewegens des Roboterarms mittels der Eingabevorrichtung ergibt sich ein relativ einfaches manuelles Bewegen des Roboterarms.

Die erfindungsgemäße Bedienvorrichtung weist neben der mechanischen Eingabevorrichtung die mit dieser gekoppelten Steuerungsvorrichtung auf. Aufgrund des Bewegens der Eingabevorrichtung erzeugt die Steuerungsvorrichtung Signale, mittels derer die Bewegung des Roboterarms entsprechend der Bewegung der Eingabevorrichtung gesteuert wird. Die Steuerungsvorrichtung kann z.B. direkt die Roboterarme ansteuern oder mit einer weiteren Steuerungsvorrichtung verbunden sein, die Antriebe zum Bewegen der Roboterarme ansteuert. Im zweiten Fall kann, wenn mehrere Roboterarme verwendet werden, eine einzige Steuerungsvorrichtung alle oder mehrere der Roboterarme gemeinsam direkt ansteuern oder es kann jedem einzelnen Roboterarm eine individuelle Steuerungsvorrichtung zugeordnet sein, die von der Steuervorrichtung der erfindungsgemäßen Bedienvorrichtung angesteuert werden.

Die Eingabevorrichtung ist dafür vorgesehen, von einer Person mit der Hand bewegt zu werden. Durch den so genannten Tremor dieser Person wird der gewollten manuellen Bewegung der Eingabevorrichtung eine durch den Tremor hervorgerufene Zitterbewegung, die resultierende Teilbewegung, überlagert. Erfindungsgemäß weist die Eingabevorrichtung jedoch wenigstens eine mechanische Dämpfungseinrichtung auf, die die Kraft bzw. das Drehmoment erzeugt, die bzw. das der manuellen Bewegung der Eingabevorrichtung entgegengerichtet ist und derart ausgeführt ist, dass es die durch den Tremor der die Eingabevorrichtung bedienenden Person resultierenden Teilbewegung zumindest teilweise unterdrückt. Die mechanische Dämpfungseinrichtung erzeugt insbesondere eine von der Bewegung der Eingabevorrichtung abhängige Kraft bzw. ein von der Bewegung der Eingabevorrichtung abhängiges Drehmoment, die bzw. das entgegen der Teilbewegung gerichtet ist.

Die mechanische Dämpfungseinrichtung kann beispielsweise ein passives viskoses Dämpfungselement aufweisen, dessen Dämpfung insbesondere einem dem Tremor zugeordneten Frequenzbereich zugeordnet ist. Dadurch wird es ermöglicht, in relativ einfacher Weise gezielt die Kraft bzw. das Drehmoment entgegen die durch den Tremor erzeugte Zitterbewegung zu richten und diese zumindest teilweise zu unterdrücken. Die Größe der Dämpfung des Dämpfungselements kann konstant sein, aber auch einstellbar sein, um z.B. an eine bestimmte, die erfindungsgemäße Bedienvorrichtung bedienende Person angepasst zu werden. Eine Einstellung der Dämpfung kann z.B. über elektrorheologische Flüssigkeiten erfolgen. Die Dämpfung kann auch abhängig von der Geschwindigkeit der manuellen Bewegung der Eingabevorrichtung und/oder abhängig von einer Skalierung sein, so dass sich der Grad der Bewegung des Roboterarms um die Skalierung von dem Grad der Bewegung der Eingabevorrichtung unterscheidet. Insbesondere ein Runterskalieren kann im medizinischen Umfeld angewendet werden, um den Roboterarm z.B. eine relativ feine Bewegung ausführen zu lassen während die die Bedienvorrichtung bedienende Person eine entsprechende relativ großräumige Bewegung durchführt. In der Chirurgie ist dies beispielsweise bei Anastomosen von Interesse.

Die Eingabevorrichtung weist erfindungsgemäß eine Achse, einen drehbar um die Achse und/oder längs der Achse verschiebbar gelagerten Hebel und einen Elektromotor auf, der eingerichtet ist, bei einer manuellen Bewegung des Hebels längs der Achse die dieser manuellen Bewegung entgegen gerichtete Kraft auf den Hebel und/oder bei einer manuellen Bewegung des Hebels um die Achse das dieser manuellen Bewegung entgegen gerichtete Drehmoment auf den Hebel zu erzeugen. Die Eingabevorrichtung weist demnach die wenigstens eine Achse auf, der der Elektromotor zugeordnet ist. Der Elektromotor kann u.A. dafür verwendet werden, die Bedienvorrichtung kraftgeregelt auszuführen. Die Kraftregelung wird insbesondere dafür verwendet, gezielt in dem Bereich, in dem die durch den Tremor entstehende Zitterbewegung (Teilbewegung) entsteht, einzuwirken, indem der Elektromotor beispielsweise derart angesteuert oder geregelt wird, um die der Zitterbewegung entgegen gerichtete Kraft bzw. das der Zitterbewegung entgegen gesetzte Drehmoment auf den der Achse zugeordneten Hebel aufzubringen. Somit ist es in relativ einfacher Weise und mit relativ einfachen Mitteln möglich, die Zitterbewegung zumindest teilweise zu kompensieren, insbesondere dann, wenn die Bedienvorrichtung kraftgeregelt ausgeführt ist.

Das vom Elektromotor aufzubringende entgegen gerichtete Drehmoment $\tau_{\text{Motor}}$ kann beispielsweise gemäß folgender Formel berechnet werden:

$$\tau_{\text{Motor}} = D \cdot (\dot{\theta}_S - \dot{\theta})$$

wobei D ein Dämpfungsfaktor, der insbesondere dem Frequenzbereich des Tremors zugeordnet ist, $\dot{\theta}$ die Geschwindigkeit ist, mit der sich der Hebel um die Achse dreht, und $\dot{\theta}_S$ die zeitliche Ableitung eines Soll-Winkels des Hebels ist. Ändert sich der Soll-Winkel nicht oder nur verhältnismäßig langsam, dann wird $\dot{\theta}_S$ null bzw. kann gegebenenfalls vernachlässigt werden. Der Dämpfungsfaktor kann konstant oder z.B. von der Geschwindigkeit abhängig sein, mit der sich der Hebel um die Achse dreht. Die Größe der Dämpfung kann auch einstellbar sein, um z.B. an eine bestimmte, die erfindungsgemäße Bedienvorrichtung bedienende Person angepasst zu werden. Die Dämpfung kann auch abhängig von einer Skalierung sein, wobei sich der Grad der Bewegung des Roboterarms um die Skalierung von dem Grad der Bewegung der Eingabevorrichtung unterscheidet.

Nach weiteren Ausführungsformen der erfindungsgemäßen Bedienvorrichtung kann das vom Elektromotor aufzubringende entgegen gerichtete Drehmoment $\tau_{\text{Motor}}$ gemäß einer der folgenden Formeln berechnet werden:

$$\tau_{\text{Motor}} = D \cdot (\dot{\theta}_S - \dot{\theta}) + k(\theta_S - \theta)$$

$$\tau_{\text{Motor}} = D \cdot (\dot{\theta}_S - \dot{\theta}) + m \cdot (\ddot{\theta}_S - \ddot{\theta})$$

$$\tau_{\text{Motor}} = D \cdot (\dot{\theta}_S - \dot{\theta}) + k(\theta_S - \theta) + m \cdot (\ddot{\theta}_S - \ddot{\theta})$$

wobei k eine dem Hebel zugeordnete virtuelle Steifigkeit, m eine dem Hebel zugeordnete virtuelle Masse bzw. Trägheit, $\theta_S$ ein Soll-Winkel des Hebels bezüglich der Achse, $\theta$ ein (Soll-)Winkel, um den der Hebel bezüglich des Soll-Winkels $\theta_S$ um die Achse gedreht ist, $\ddot{\theta}$ die Beschleunigung ist, mit der sich der Hebel um die Achse dreht, und $\dot{\theta}_S$ die zeitliche Ableitung des Soll-Winkels des Hebels ist. Ändert sich der Soll-Winkel nicht oder nur verhältnismäßig langsam, dann wird $\dot{\theta}_S$ und $\ddot{\theta}_S$ null bzw. kann gegebenenfalls vernachlässigt werden.

Die Geschwindigkeit kann z.B. mittels dem Hebel zugeordneter Positionssignalen ermittelt werden. Bei der Wahl des Dämpfungsfaktors und gegebenenfalls der virtuellen Steifigkeit und der virtuellen Masse/ der virtuellen Trägheit kann eine durch die Berechnung der Geschwindigkeit mittels der Positionssignale entstehende Latenz berücksichtigt werden. Die Geschwindigkeit kann aber auch mittels eines dem Hebel bzw. der Achse zugeordneten Geschwindigkeitssensors ermittelt werden.

Ausführungsbeispiele der Erfindung sind exemplarisch in den beigefügten schematischen Zeichnungen dargestellt. Es zeigen:

Fig. 1 einen medizinischen Arbeitsplatz mit mehreren Roboterarmen und eine mechanische Handeingabevorrichtungen aufweisende Bedienvorrichtung zum manuellen Bewegen der Roboterarme und

Fig. 2 und 3 Detailansichten einer der mechanischen Handeingabevorrichtungen und

Fig. 4 eine Detailansicht einer alternativen mechanischen Handeingabevorrichtung für die Bedienvorrichtung.

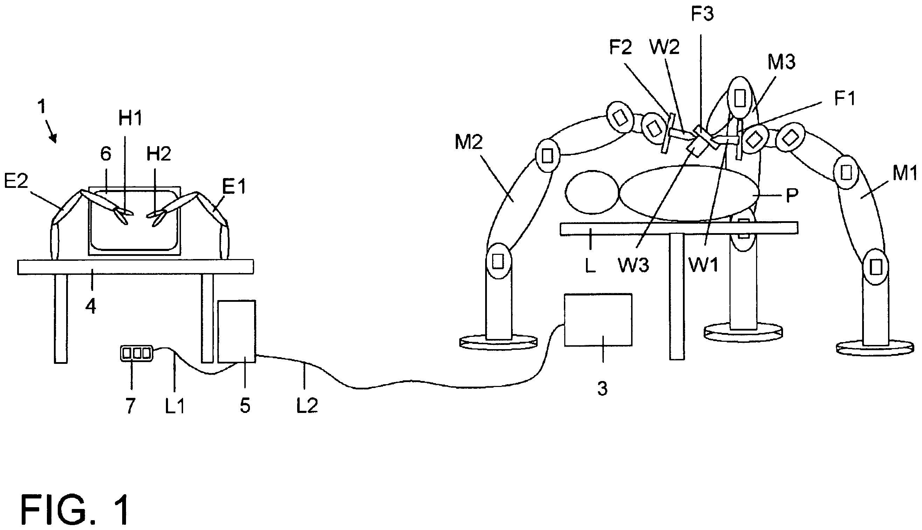

Die Fig. 1 zeigt einen medizinischen Arbeitsplatz, der eine Patientenliege L, mehrere Roboterarme M1-M3 und eine Bedienvorrichtung 1 zum manuellen Bewegen der Roboterarme M1-M3 aufweist. Jeder der Roboterarme M1-M3 weist mehrere mittels Antrieben bewegbare Achsen und eine Befestigungsvorrichtung F1-F3 auf und kann beispielsweise bezüglich sechs Freiheitsgraden bewegt werden.

Im Falle des vorliegenden Ausführungsbeispiels liegt auf der Patientenliege L eine Person P, die mittels der Roboterarme M1-M3 bzw. mittels an den Befestigungsvorrichtungen F1-F3 der Roboterarme M1-M3 befestigten Instrumenten behandelt werden kann. An den Befestigungsvorrichtungen F1, F2 der Roboterarme M1, M2 sind z.B. jeweils medizinische Instrumente W1, W2 und an der Befestigungsvorrichtung F3 des Roboterarms M3 ist beispielsweise eine Kamera W3 befestigt.

Im Falle des vorliegenden Ausführungsbeispiels sind die Antriebe der Roboterarme M-M3, die medizinischen Instrumente W1, W2 und die Kamera W3 in nicht dargestellter Weise mit einem ersten Steuerrechner 3 verbunden. Auf dem ersten Steuerrechner 3 läuft ein Rechnerprogramm, mittels dem der Steuerrechner 3 die Antriebe der Roboterarme M1-M3 derart ansteuern kann, so dass sich die Achsen der Roboterarme M1-M3 in gewünschter Weise derart bewegen, damit die Befestigungsvorrichtungen F1-F3 bzw. die Tool Center Points der medizinischen Instrumente W1, W2 und der Kamera W3 eine gewünschte Lage (Position und Orientierung) einnehmen.

Der medizinische Arbeitsplatz umfasst ferner die Bedienvorrichtung 1. Diese weist im Falle des vorliegenden Ausführungsbeispiels einen zweiten Steuerrechner 5, zwei an einem Tisch 4 angeordnete und in nicht dargestellter Weise mit dem zweiten Steuerrechner 5 verbundene Handeingabevorrichtungen E1, E2, einen in nicht dargestellter Weise mit dem zweiten Steuerrechner 5 verbundenen Bildschirm 6 und eine mittels einer Leitung L1 mit dem zweiten Steuerrechner 5 verbundene Fußumschalteinrichtung 7 auf. Die beiden Steuerrechner 3, 5 können ferner über eine Datenleitung L2 miteinander kommunizieren.

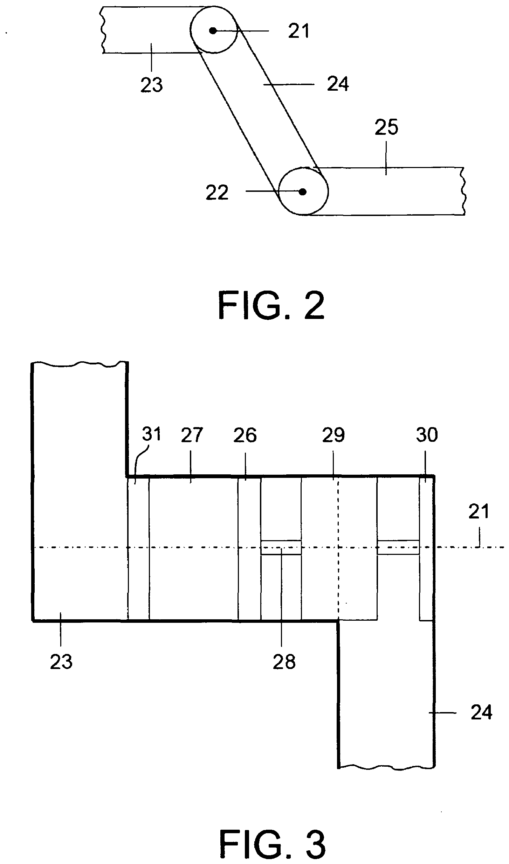

Ein Teil einer der beiden Handeingabevorrichtungen E1, E2, z.B. der Handeingabevorrichtung E1 ist in den Figuren 2 und 3 dargestellt.

Im Falle des vorliegenden Ausführungsbeispiels weisen die beiden Handeingabevorrichtungen E1, E2 jeweils einen Handgriff H1, H2, mehrere Achsen 21, 22 und mehrere Hebel 23-25 auf. In der in der Fig. 2 gezeigten Detailansicht der Handeingabevorrichtung E1 ist der Hebel 24 relativ zum Hebel 23 drehbar um die Achse 21 und der Hebel 25 relativ zum Hebel 24 drehbar um die Achse 22 gelagert dargestellt.

Ein in den Figuren nicht näher dargestellter Arzt kann die Handeingabevorrichtungen E1, E2 mittels der Handgriffe H1, H2 manuell bezüglich wenigstens sechs Freiheitsgraden bewegen. Die Handeingabevorrichtungen E1, E2 weisen z.B. den jeweiligen Achsen 21, 22 der Handeingabevorrichtungen E1, E2 zugeordnete und in der Fig. 3 dargestellte Winkelsensoren 26 auf, deren Signale dem zweiten Steuerrechner 5 übermittelt werden. Auf dem zweiten Steuerrechner 5 läuft wiederum ein Rechnerprogramm, das aufgrund der von den Handeingabevorrichtungen E1, E2 stammenden Signalen Bewegungen der Handeingabevorrichtungen E1, E2 erkennt. Die beiden Handeingabevorrichtungen E1, E2 können auch mehr als sechs Freiheitsgrade aufweisen.

Im Falle des vorliegenden Ausführungsbeispiels ist die Handeingabevorrichtung E1 dafür vorgesehen, den Roboterarm M1 zu bewegen. Bei einem manuellen Bewegen der Handeingabevorrichtung E1 mittels ihres Handgriffs H1 detektieren die Winkelsensoren 26 der Handeingabevorrichtung E1 Winkeländerungen der relevanten Achsen 21, 22. Aus den von den Winkelsensoren 26 erzeugten Signalen ermittelt der zweite Steuerrechner 5 entsprechende Bewegungen der Handeingabevorrichtung E1 und übermittelt über die Leitung L2 eine entsprechende Information an den ersten Steuerrechner 3, der daraufhin die Antriebe des Roboterarms M1 derart ansteuert, so dass dessen Befestigungsvorrichtung F1 bzw. der Tool Center Point des medizinischen Instrumentes W1 eine der manuellen Bewegung der Handeingabevorrichtung E1 entsprechenden Bewegung durchführt.

Im Falle des vorliegenden Ausführungsbeispiels ist die Handeingabevorrichtung E2 dafür vorgesehen, die beiden anderen Roboterarme M2, M3 zu bewegen. Um einen der beiden Roboterarme M2, M3 für das Bewegen auszuwählen, kann der Arzt die Fußumschalteinrichtung 7 betätigen, der mit der Steuerleitung L1 mit dem zweiten Steuerrechner 5 verbunden ist.

Im Falle des vorliegenden Ausführungsbeispiels ist am Roboterarm M3 die Kamera W3 befestigt, mit der Bilder vom Operationssitus aufgenommen werden können, so dass der Arzt z.B. eine optische Rückmeldung über die Lagen der Roboterarme M1, M2 und/oder der medizinischen Instrumente W1, W2 erhält. Die den mit der Kamera W3 aufgenommenen Bildern zugeordneten Bilddatensätze werden über die Leitung L2 vom ersten Steuerrechner 3 zum zweiten Steuerrechner 5 übermittelt, damit der zweite Steuerrechner 5 diese Bilder am Bildschirm 6 darstellten kann.

Personen weisen in der Regel eine natürlich Zitterbewegung, den so genannten Tremor, auf. Aufgrund des Tremors wird einer gewollten Bewegung des Arztes beim Bewegen der Handeingabevorrichtungen E1, E2 eine der Zitterbewegung zugeordnete Teilbewegung überlagert. Um diese zumindest teilweise zu unterdrücken, umfassen die Handeingabevorrichtungen E1, E2 Dämpfungseinrichtungen.

Im Falle des vorliegenden Ausführungsbeispiels sind z.B. der Achse 21 bzw. dem Hebel 24, der wie in der Fig. 3 näher dargestellt z.B. mittels einer Lagerung 30 bezüglich der Achse 21 relativ zum Hebel 23 drehbar gelagert ist, ein Elektromotor 27 zugeordnet, der mittels seiner Welle 28 mit einem Getriebe 29 gekoppelt ist. Der Elektromotor 27 weist z.B. auch eine Bremse 31 auf. Der Elektromotor 27, die Bremse 31 und der Winkelsensor 26 sind in nicht dargestellter Weise mit dem zweiten Steuerrechner 5 verbunden. Den weiteren Achsen der Handeingabevorrichtungen E1, E2 können ebenfalls Elektromotoren zugeordnet sein.

Mittels des Elektromotors 27 kann auf den Hebel 24 eine Kraft bzw. ein Drehmoment $\tau_{Motor}$ aufgebracht werden. Auf dem zweiten Steuerrechner 5 läuft nicht nur ein Rechnerprogramm, das aufgrund von den Winkelsensoren 26 stammenden Signalen die Signale zum Bewegen der Roboterarme M1-M3 erzeugt, sondern auch ein Rechnerprogramm, das den Elektromotor 27 derart ansteuert oder regelt, so dass dieser ein der Zitterbewegung des die Bedienvorrichtung 1 bedienenden Arztes entgegen gesetztes Drehmoment $\tau_{Motor}$ erzeugt. Dadurch wird die die gewollte Bewegung des Hebels 24 überlagerte Zitterbewegung gedämpft, wodurch durch die Zitterbewegung des Arztes verursachten Signalkomponenten der von den Winkelsensoren 26 stammenden Signalen zumindest reduziert werden.

Im Falle des vorliegenden Ausführungsbeispiels berechnet der zweite Steuerrechner 5 das vom Elektromotor 27 aufzubringende Drehmoment $\tau_{Motor}$ gemäß folgender Gleichung:

$$\tau_{Motor} = D \cdot (\dot{\theta}_S - \dot{\theta})$$

wobei D ein Dämpfungsfaktor, $\dot{\theta}$ die Geschwindigkeit ist, mit der sich der Hebel 24 um die Achse 21 dreht, und $\dot{\theta}_S$ die zeitliche Ableitung eines Soll-Winkels des Hebels 24 ist. Ändert sich der Soll-Winkel nicht oder nur verhältnismäßig langsam, dann wird $\dot{\theta}_S$ null bzw. kann gegebenenfalls vernachlässigt werden.

Die Geschwindigkeit, mit der sich der Hebel 24 aufgrund der manuellen Bewegung um die Achse 21 dreht, berechnet im Falle des vorliegenden Ausführungsbeispiels der zweite Steuerrechner 5 aus den von dem Winkelsensor 26 stammenden Signalen. Andere Verfahren zum Ermitteln dieser Geschwindigkeit, wie z.B. eine direkte Messung mittels eines Geschwindigkeitssensors, sind auch möglich.

Der Dämpfungsfaktor kann konstant sein, ist aber im Falle des vorliegenden Ausführungsbeispiels abhängig von der Geschwindigkeit, mit der der Hebel 24 um die Achse 21 gedreht wird, abhängig von einer Skalierung und/oder abhängig von der Person, die die Bedienvorrichtung 1 betätigt. Bei einer Skalierung unterscheidet sich der Grad der Bewegung des Roboterarms M1-M3 um die Skalierung von dem Grad der Bewegung der Handeingabevorrichtung E1, E2.

Der zweite Steuerrechner 5 kann das Drehmoment $\tau_{Motor}$ für den Elektromotor 27 auch gemäß einer der folgenden Formeln berechnen:

$$\tau_{Motor} = D \cdot (\dot{\theta}_S - \dot{\theta}) + k(\theta_S - \theta)$$

$$\tau_{Motor} = D \cdot (\dot{\theta}_S - \dot{\theta}) + m \cdot (\ddot{\theta}_S - \ddot{\theta})$$

$$\tau_{Motor} = D \cdot (\dot{\theta}_S - \dot{\theta}) + k(\theta_S - \theta) + m \cdot (\ddot{\theta}_S - \ddot{\theta})$$

wobei k eine dem Hebel 24 zugeordnete virtuelle Steifigkeit, m eine dem Hebel 24 zugeordnete virtuelle Masse bzw. Trägheit, $\theta_S$ ein Soll-Winkel des Hebels 24 bezüglich der Achse 21, $\theta$ der Winkel, um den der Hebel 24 bezüglich des Soll-Winkels $\theta_S$ um die Achse 21 gedreht ist, und $\ddot{\theta}$ die Beschleunigung, mit der sich der Hebel 24 um die Achse 21 dreht.

Im Falle des in den Figuren 2 und 3 gezeigten Ausführungsbeispiels ist der Hebel 24 drehbar um die Achse 21 gelagert. Es ist aber auch möglich, dass die Handeingabevorrichtungen E1, E2 einen oder mehrere Hebel aufweisen, der längs einer Achse verschieblich gelagert ist. Ist z.B. der Hebel 24 längs der Achse 21 verschieblich gelagert ausgeführt, dann kann der Elektromotor 27 eine translatorische Bewegung des Hebels 24 längs der Achse 21 derart dämpfen, so dass die durch die Zitterbewegung des Arztes erzeugte Teilbewegung zumindest teilweise unterdrückt wird.

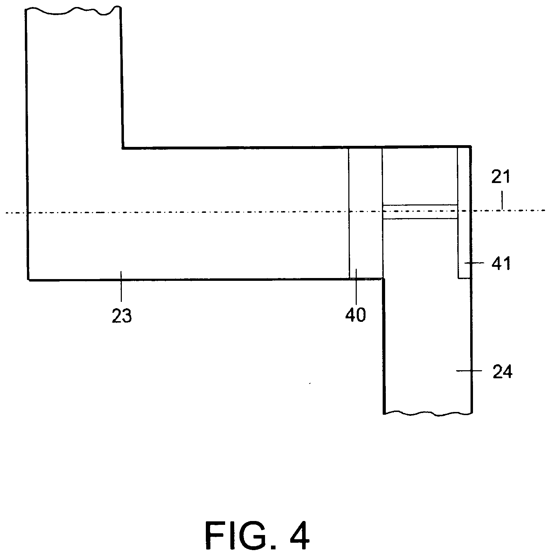

Die Fig. 4 zeigt ein weiteres Ausführungsbeispiel einer Dämpfungseinrichtung für die Handeingabevorrichtungen E1, E2, die zusätzlich oder alternativ zu der als Elektromotor 27 ausgebildeten Dämpfungseinrichtung verwendet werden kann.

Im Falle der in der Fig. 4 dargestellten Dämpfungseinrichtung handelt es sich um ein viskoses Dämpfungselement 40, das mittels einer Lagerung 41 am Hebel 24 befestigt ist und eine Bewegung des Hebels 24 um die Achse 21 insbesondere in einem dem Tremor des Arztes zugeordneten Frequenzbereich dämpft.

Der Dämpfungsfaktor des Dämpfungselements 40 kann konstant sein, ist aber im Falle des vorliegenden Ausführungsbeispiels abhängig von der Geschwindigkeit, mit der der Hebel 24 um die Achse 21 gedreht wird, abhängig von einer Skalierung und/oder abhängig von der Person, die die Bedienvorrichtung 1 betätigt.

Im Falle des in der Fig. 4 gezeigten Ausführungsbeispiels ist der Hebel 24 drehbar um die Achse 21 gelagert. Es ist aber auch möglich, dass die Handeingabevorrichtungen E1, E2 einen oder mehrere Hebel aufweisen, der längs einer Achse verschieblich gelagert ist. Ist z.B. der Hebel 24 längs der Achse 21 verschieblich gelagert ausgeführt, dann kann das Dämpfungselement 40 eine translatorische Bewegung des Hebels 24 längs der Achse 21 derart dämpfen, so dass die durch die Zitterbewegung des Arztes erzeugte Teilbewegung zumindest teilweise unterdrückt wird.

**Patentansprüche**

1. Bedienvorrichtung zum manuellen Bewegen eines Roboterarms, aufweisend

    - eine Steuerungsvorrichtung (5), die eingerichtet ist, zum Steuern einer Bewegung wenigstes eines zum Behandeln eines Lebewesens (P) vorgesehenen Roboterarms (M1-M3) vorgesehene Signale zu erzeugen, und
    - wenigstens eine mit der Steuerungsvorrichtung (5) gekoppelte manuelle mechanische Eingabevorrichtung (E1-E3), die eine Achse (21), einen drehbar um die Achse (21) und/oder längs der Achse (21) verschiebbar gelagerten Hebel (24) aufweist, wobei die Steuerungsvorrichtung (5) die Signale aufgrund eines manuellen Bewegens der Eingabevorrichtung (E1-E3) erzeugt, so dass der Roboterarm (M1-M3) eine der manuellen Bewegung entsprechenden Bewegung durchführt, **dadurch gekennzeichnet, dass** die Eingabevorrichtung (E1, E2) wenigstens eine mechanische Dämpfungseinrichtung umfasst, die bei einem manuellen Bewegen der Eingabeeinrichtung (E1, E2) eine Kraft und/oder ein Drehmoment zum zumindest teilweisen Unterdrücken einer durch einen Tremor der die Eingabevorrichtung (E1, E2) bedienenden Person resultierenden Teilbewegung erzeugt, und die Eingabevorrichtung (E1, E2) dafür einen Elektromotor (27) aufweist,

    der eingerichtet ist, bei einer manuellen Bewegung des Hebels (24) längs der Achse (21) die dieser manuellen Bewegung entgegen gerichtete Kraft auf den Hebel (24) und/oder bei einer manuellen Bewegung des Hebels (24) um die Achse (21) das dieser manuellen Bewegung entgegen gerichtete Drehmoment auf den Hebel (24) zu erzeugen.

2. Bedieneinrichtung nach Anspruch 1, bei der die mechanische Dämpfungseinrichtung ein passives viskoses Dämpfungselementvorrichtung (40) aufweist, dessen Dämpfung insbesondere einstellbar, abhängig von der Geschwindigkeit der manuellen Bewegung der Eingabevorrichtung (E1, E2), einem dem Tremor zugeordneten Frequenzbereich zugeordnet ist und/oder abhängig von einer Skalierung ist, so dass sich der Grad der Bewegung des Roboterarms um die Skalierung von dem Grad der Bewegung der Eingabevorrichtung unterscheidet.

3. Bedienvorrichtung nach Anspruch 1 oder 2, bei der das vom Elektromotor (27) aufzubringende entgegen gerichtete Drehmoment gemäß folgender Formel berechnet wird:

$$\tau_{Motor} = D \cdot (\dot{\theta}_S - \dot{\theta})$$

wobei D ein Dämpfungsfaktor, der insbesondere dem Frequenzbereich des Tremors zugeordnet ist, $\dot{\theta}$ die Geschwindigkeit ist, mit der sich der Hebel (24) um die Achse (21) dreht, und $\theta_S$ die zeitliche Ableitung eines Soll-Winkels des Hebels (24) ist.

4. Bedienvorrichtung nach Anspruch 1 oder 2, bei der das vom Elektromotor (27) auf zubringende entgegen gerichtete Drehmoment gemäß einer der folgenden Formeln berechnet wird:

$$\tau_{Motor} = D \cdot (\dot{\theta}_S - \dot{\theta}) + k(\theta_S - \theta)$$

$$\tau_{Motor} = D \cdot (\dot{\theta}_S - \dot{\theta}) + m \cdot (\ddot{\theta}_S - \ddot{\theta})$$

$$\tau_{Motor} = D \cdot (\dot{\theta}_S - \dot{\theta}) + k(\theta_S - \theta) + m \cdot (\ddot{\theta}_S - \ddot{\theta})$$

wobei D ein Dämpfungsfaktor, der insbesondere dem Frequenzbereich des Tremors zugeordnet ist, k eine dem Hebel zugeordnete virtuelle Steifigkeit, m eine dem Hebelzugeordnete virtuelle Masse bzw. Trägheit, $\theta_S$ ein Soll-Winkel des Hebels (24) bezüglich der Achse (21), $\theta$ ein Winkel, um den der Hebel (24) bezüglich des Soll Winkels $\theta_S$ um die Achse (21) gedreht ist, $\dot{\theta}$ die Geschwindigkeit, mit der sich der Hebel (24) um die Achse (21) dreht, und $\ddot{\theta}$ die Beschleunigung ist, mit der sich der Hebel (24) um die Achse (21) dreht.

5. Bedienvorrichtung nach Anspruch 3 oder 4, bei der der Dämpfungsfaktor von der Geschwindigkeit abhängt, mit der sich der Hebel (24) um die Achse (21) dreht.

6. Medizinischer Arbeitsplatz, aufweisend eine Bedienvorrichtung (1) nach einem der Ansprüche 1 bis 5 und wenigstes einen zum Behandeln des Lebewesens (P) vorgesehenen medizinischen Roboterarm (M1-M3), dessen Bewegung mittels der Bedienvorrichtung (1) manuell steuerbar ist.

**Claims**

1. Operating device for the manual movement of a robot arm, comprising

   - a controller (5) which is configured to generate signals for controlling a movement of at least one robot arm (M1-M3) provided for treating a living being (P), and
   - at least one manual mechanical input device (E1-E3) coupled to the controller (5), which input device comprises an axis (21), a lever (24) mounted to move rotatably about the axis (21) and/or along the axis (21), wherein the controller (5) produces the signals on the basis of a manual movement of the input device (EI-E3) so that the robot arm (M1-M3) performs a movement corresponding to the manual movement, **characterised in that** the input device (E1, E2) comprises at least one mechanical damping device, which during a manual movement of the input device (E1, E2) produces a force and/or a torque for at least partly suppressing a partial movement resulting from a tremor of the person operating the input device (E1, E2), and the input device (E1, E2) comprises an electric motor (27) for this, which is configured during a manual movement of the lever (24) along the axis (21) to produce the force directed against said manual movement on the lever (24) and/or during a manual movement of the lever (24) about the axis (21) to produce the torque directed against said manual movement on the lever (24).

2. Operating device according to claim 1, wherein the mechanical damping device comprises a passive viscous damping element device (40), the damping of which is assigned in particular adjustably, based on the speed of the manual movement of the input device (E1, E2), to a frequency range assigned to the tremor and/or is based on scaling, so that the degree of movement of the robot arm differs from the degree of movement of the input device by the scaling factor.

3. Operating device according to claim 1 or 2, wherein the opposing torque applied by the electric motor (27) is calculated according to the following formula:

$$\tau_{Motor} = D \cdot (\dot{\theta}_S - \dot{\theta})$$

wherein D is a damping factor, which is assigned in particular to the frequency range of the tremor, $\dot{\theta}$ is the speed at which the lever (24) rotates about the axis (21), and $\dot{\theta}_S$ is the time derivative of a set angle of the lever (24).

4. Operating device according to claim 1 or 2, wherein the opposing torque applied by the electric motor (27) is calculated according to one of the following formulae:

$$\tau_{Motor} = D \cdot (\dot{\theta}_S - \dot{\theta}) + k(\theta_S - \theta)$$

$$\tau_{Motor} = D \cdot (\dot{\theta}_S - \dot{\theta}) + m \cdot (\ddot{\theta}_S - \ddot{\theta})$$

$$\tau_{Motor} = D \cdot (\dot{\theta}_S - \dot{\theta}) + k(\theta_S - \theta) + m \cdot (\ddot{\theta}_S - \ddot{\theta})$$

wherein D is a damping factor, which in particular is assigned to the frequency range of the tremor, k is a virtual rigidity assigned to the lever, m is a virtual mass or inertia assigned to the lever, $\dot{\theta}_S$ is a set angle of the lever (24) relative to the axis (21), $\theta$ is an angle by which the lever (24) is rotated relative to the set angle $\theta_S$ about the axis (21), $\dot{\theta}$ is the speed at which the lever (24) rotates about the axis (21), and $\ddot{\theta}$ is the acceleration at which the lever (24) rotates about the axis (21).

5.  Operating device according to claim 3 or 4, wherein the damping factor is dependent on the speed at which the lever (24) rotates about the axis (21).

6.  Medical workstation, comprising an operating device (1) according to any of claims 1 to 5 and at least one medical robot arm (M1-M3) provided for treating the living being (P), the movement of which can be controlled manually by means of the operating device (1).


**Revendications**

1.  Dispositif de manutention pour le déplacement manuel d'un bras de robot, comportant

    - un dispositif de commande (5) qui est conçu pour engendrer des signaux prévus pour commander un déplacement d'au moins un bras de robot (M1-M3) prévu pour le traitement d'un être vivant (P), et
    - au moins un dispositif d'entrée (E1-E3) mécanique manuel accouplé au dispositif de commande (5), lequel présente un axe (21), un levier (24) monté de façon rotative autour de l'axe (21) et/ou mobile le long de l'axe (21), le dispositif de commande (5) engendrant les signaux sur la base d'un déplacement manuel du dispositif d'entrée (E1-E3), de telle sorte que le bras de robot (M1-M3) exécute un déplacement correspondant au déplacement manuel, **caractérisé en ce que** le dispositif d'entrée (E1, E2) comprend au moins un moyen d'amortissement mécanique qui, lors d'un déplacement manuel du dispositif d'entrée (E1, E2), engendre une force et/ou un couple pour supprimer au moins partiellement un mouvement partiel résultant d'un tremblement de la personne manipulant le dispositif d'entrée (E1, E''), et **en ce que** le dispositif d'entrée (E1, E2) présente à cet effet un moteur électrique (27) qui est conçu pour engendrer, lors d'un déplacement manuel du levier (24) le long de l'axe (21), la force exercée sur le levier (24) et dirigée en sens inverse de ce déplacement manuel, et/ou pour engendrer, lors d'un déplacement manuel du levier (24) autour de l'axe (21), le couple exercé sur le levier (24) et dirigé en sens inverse de ce déplacement manuel.

2.  Dispositif de manutention selon la revendication 1, dans lequel le moyen d'amortissement mécanique présente un dispositif d'élément amortisseur (40) visqueux passif dont l'amortissement est en particulier réglable en fonction de la vitesse du déplacement manuel du dispositif d'entrée (E1, E2), est associé à une plage de fréquence qui est associée au tremblement et/ou dépend d'une graduation, de telle sorte que le degré du déplacement du bras de robot se distingue du degré du déplacement du dispositif d'entrée, de la valeur de la graduation.

3.  Dispositif de manutention selon les revendications 1 ou 2, dans lequel le couple dirigé en sens inverse, qui doit être appliqué par le moteur électrique, est calculé selon la formule suivante :

$$\tau_{Motor} = D \cdot (\dot{\theta}_S - \dot{\theta})$$

D étant un facteur d'amortissement qui est en particulier associé à la plage de fréquence du tremblement, $\dot{\theta}$ étant la vitesse à laquelle le levier (24) tourne autour de l'axe (21), et $\dot{\theta}_S$ étant la dérivée de temps d'un angle de consigne du levier (24).

**4.** Dispositif de manutention selon les revendications 1 ou 2, dans lequel le couple dirigé en sens inverse, qui doit être appliqué par le moteur électrique, est calculé selon l'une des formules suivantes :

$$\tau_{Motor} = D \cdot (\dot{\theta}_S - \dot{\theta}) + k(\theta_S - \theta)$$

$$\tau_{Motor} = D \cdot (\dot{\theta}_S - \dot{\theta}) + m \cdot (\ddot{\theta}_S - \ddot{\theta})$$

$$\tau_{Motor} = D \cdot (\dot{\theta}_S - \dot{\theta}) + k(\theta_S - \theta) + m \cdot (\ddot{\theta}_S - \ddot{\theta})$$

D étant un facteur d'amortissement qui est en particulier associé à la plage de fréquence du tremblement, k étant une rigidité virtuelle associée au levier, m étant une masse ou une inertie virtuellement associée au levier, $\theta_S$ étant un angle de consigne du levier (24) par rapport à l'axe (21), $\theta$ étant un angle autour duquel le levier (24) est tourné autour de l'axe (21) par rapport à l'angle de consigne $\theta_S$, $\dot{\theta}$ étant la vitesse à laquelle le levier (24) tourne autour de l'axe (21), et $\ddot{\theta}$ étant l'accélération avec laquelle le levier (24) tourne autour de l'axe (21).

**5.** Dispositif de manutention selon les revendications 3 ou 4, dans lequel le facteur d'amortissement dépend de la vitesse à laquelle le levier (24) tourne autour de l'axe (21).

**6.** Poste de travail médical présentant un dispositif de manutention (1) selon l'une des revendications 1 à 5 et au moins un bras de robot (M1-M3) médical prévu pour le traitement de l'être vivant (P), dont le déplacement peut être commandé manuellement au moyen du dispositif de manutention (1).

FIG. 1

EP 2 326 275 B1

# FIG. 2

# FIG. 3

# FIG. 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0883376 B1

- WO 2007005367 A2